(19) Europäisches Patentamt
European Patent Office
Office européen des brevets

(11) Publication number: 0 015 603
B1

(12) EUROPEAN PATENT SPECIFICATION

(45) Date of publication of patent specification: 02.02.83

(51) Int. Cl.$^3$: C 07 C 2/22, C 07 C 2/34, B 01 J 31/22, C 08 F 4/76

(21) Application number: 80200139.6

(22) Date of filing: 19.02.80

# (54) Process for the dimerization of 1-alkenes.

(30) Priority: 02.03.79 GB 7907546

(43) Date of publication of application:
17.09.80 Bulletin 80/19

(45) Publication of the grant of the patent:
02.02.83 Bulletin 83/5

(84) Designated Contracting States:
CH DE FR GB IT NL

(56) References cited:
DE - A - 2 318 953
DE - B - 1 442 528
GB - A - 1 447 812

(73) Proprietor: SHELL INTERNATIONALE RESEARCH MAATSCHAPPIJ B.V.
Carel van Bylandtlaan 30
NL-2596 HR Den Haag (NL)

(72) Inventor: Goodall, Brian Leslie
Badhuisweg 3
NL-1031 CM Amsterdam (NL)
Inventor: van der Heijden, Harry
Badhuisweg 3
NL-1031 CM Amsterdam (NL)

(74) Representative: Puister, Antonius Tonnis, Mr. et al,
P.O. Box 302
NL-2501 CH The Hague (NL)

EP 0 015 603 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Process for the dimerization of 1-alkenes

The invention relates to a process for the catalytic dimerization in solution of one or more alpha,beta-unsubstituted 1-alkenes having at least three carbon atoms per molecule. The invention also relates to products obtained by applying such a process.

Various processes are known for the dimerization of alkenes, such as those described in British Patent Specifications 1,058,680 and 1,182,515. However, in these patent specifications the formation of dimers is described which normally consist of mixtures of the various isomers possible, while mostly also substantial amounts of higher polymers are formed. It would be highly desirable to have a process which produces predominantly one single dimer with no or virtually no higher polymers. Although the dimerization of propylene as described in British Patent Specification 840,028 appears to be rather selective it requires rather severe reaction conditions, viz. temperatures of about 250°C and pressures ranging from 7000 to 12500 kPa.

It has now been found that alpha,beta-unsubstituted 1-alkenes having at least three carbon atoms per molecule can be dimerized highly selectively under moderate process conditions in the presence of a dimerization catalyst comprising a certain type of Lewis base. Thus, propylene, for instance, can be dimerized to 2-methyl-1-pentene.

Accordingly, the invention provides a process for the catalytic dimerization in solution of one or more alpha,beta-unsubstituted 1-alkenes having at least three carbon atoms per molecule, which is characterized in that a catalyst is used comprising a titanium component of the formula $Cp_2TiXY$, an alkylaluminium halide and a nitrogen Lewis base, in which formula Cp represents an unsubstituted or alkyl-substituted cyclopentadienyl group, X a halogen atom or an alkyl group and Y a halogen atom. The term "nitrogen Lewis base" is meant to be a Lewis base deriving its basicity from the presence of one or more nitrogen atoms having unshared electron pairs.

For the sake of completeness reference is made to GB—PS 1,447,812, in which a process is described for producing 1-butene by dimerizing ethylene in an organic solvent using a complex catalyst comprising an alkylaluminium compound and dicyclopentadienyltitaniumdichloride. However, the document is silent on the dimerization of alkenes other than ethylene.

In the present process, the dimerization of the 1-alkenes having at least three carbon atoms per molecule can be represented by the equation:

$$2R\text{—}\underset{\beta}{CH}\text{=}\underset{\alpha}{CH_2} \rightarrow R\text{—}CH_2\text{—}CH_2\text{—}\underset{\displaystyle R}{\underset{|}{C}}\text{=}CH_2$$

The process of the invention is particularly useful for the dimerization of alpha,beta-unsubstituted 1-alkenes having 3—25 carbon atoms and is preferred for those having 3—10 carbon atoms. Examples of linear 1-alkenes which can be suitably dimerized by the present process comprise propylene, 1-butene, 1-pentene, etc., examples of branched 1-alkenes are 3-methyl-1-butene, 3-methyl-1-pentene, 4-methyl-1-pentene, etc.

Titanium components preferred in the catalyst are those in whose formula $Cp_2TiXY$ both X and Y represent halogen, especially chlorine. When X represents an alkyl group, preference is given to components having not more than five carbon atoms in the alkyl group in order to preserve sufficient catalytic activity and in whose formula Y represents a chlorine atom. The cyclopentadienyl group may be substituted with one or more alkyl groups, but they do not contribute substantially to the catalytic activity. Best results have been achieved using dicyclopentadienyltitanium dichloride as the titanium component.

Suitable alkylaluminium halides comprise alkylaluminium dihalides, such as methyl- and ethylaluminium dichloride, trialkyldi-aluminium trihalides, such as trimethyl- and triethyldialuminium trichloride, as well as dialkylaluminium halides, such as dimethyl- and diethylaluminium chloride. Ethyl components are generally preferred over methyl components, whilst special preference is given to the use of ethylaluminium dichloride.

It has been found that the presence of a nitrogen Lewis base as defined above in the catalyst is of great significance for the selective dimerization of alpha,beta-unsubstituted 1-alkenes. It appears that the presence of such a Lewis base substantially prevents or reduces the formation of higher polymers during the dimerization process. In the absence of such a Lewis base a significant conversion of the starting material into higher polymeric material is likely to occur. Any Lewis bases as defined hereinbefore which are soluble in the reaction medium can be conveniently applied, such as amines and pyridine-type compounds. In particular tertiary amines are suitable, examples of which are triethylamine, triethylenediamine, dimethylcyclohexylamine and benzyldimethylamine. Special preference is given to 1,4-diazabicyclo[2.2.2]octane.

The amount of catalyst is not critical, but in general the reaction proceeds faster at higher catalyst concentrations. Although very small amounts of titanium component, e.g. 0.05 mmol. per mol. of alpha,beta-unsubstituted 1-alkene, can be suitably used in the catalyst, it is preferred to use at least 0.5 mmol. per mol. of starting material which will compensate for any decrease in catalytic activity which might be caused by reduction of the titanium under the prevailing process conditions. Normally the dimerization is carried out using the alkylaluminium halide in a fair excess compared with the titanium component, e.g., using aluminium/titanium molar ratios of 4 or more. At very high aluminium/titanium ratios the alkylaluminium halide might exert some reduction activity on the titanium. Therefore, molar ratios exceeding 20 are not used generally, preference being given to ratios between 10 and 15. The nitrogen Lewis base is usually applied in such an amount that the nitrogen/aluminium molar ratio is higher than 0.1. In order to gain full benefit from the presence of the Lewis base it is usually not necessary to employ nitrogen/aluminium molar ratios higher than 1. Generally, this ratio is preferably taken in the range of from 0.2 to 0.5.

The process of the present invention is carried out in solution. Various solvents which are inert in relation to the catalyst may be used, such as aromatic hydrocarbons or saturated aliphatic hydrocarbons, or aliphatic or aromatic ethers. Best results are obtained when using such solvents which optimally dissolve the catalyst and have no tendency to react with alpha,beta-unsubstituted 1-alkenes, such as halogenated aromatic hydrocarbons, in particular bromobenzene and chlorobenzene.

The process of the present invention can be carried out suitably at temperatures up to 80°C, preferably between 10 and 40°C, although lower and higher temperatures may be used as long as the various components present in the reaction mixture remain in solution.

The process can be carried out at atmospheric or super-atmospheric pressure. Especially when gaseous or low-boiling alpha,beta-unsubstituted 1-alkenes are used as starting materials, superatmospheric pressures can be advantageously applied. Pressures up to 1000 kPa can be suitably applied, while preference is given to pressure between 100 and 500 kPa depending on the starting material employed.

The process is carried out under exclusion of air and moisture in order to prevent degradation of the catalyst. The alpha,beta-unsubstituted 1-alkene as such may be sufficient to achieve this, but the additional presence of an inert gas, such as nitrogen, helium or argon, may be required.

The term "dimerization" used in connection with the process of the invention is also applicable to similar reactions taking place in a mixture of more than one alpha,beta-unsubstituted 1-alkene. It will be appreciated that the presence of two or more of such 1-alkenes may lead to the additional formation of "co-dimers" by combination of different monomers in addition to the formation of the specific dimers expected to be formed.

The dimers prepared by the present process can be used for various outlets, for example as intermediates for detergents or as high-octane gasoline additives.

The invention is illustrated by the following Examples.

Example 1

In a 30 ml glass bottle, placed in a nitrogen atmosphere, 10 mg (0.04 mmol.) of dicyclopentadienyltitanium dichloride ($Cp_2TiCl_2$) were weighed in, followed by 13 mg (0.12 mmol.) of 1,4-diaza-bicyclo[2.2.2]octane, after which 15 ml of bromobenzene were added. The resulting solution was saturated with propylene, whereupon 0.062 ml (0.6 mmol.) of ethylaluminium dichloride was added to the solution. The propylene pressure was then raised to 200 kPa. The temperature was kept on 25°C.

Gas-chromatographic analysis of the product revealed that substantially one product had been formed, which appeared to be 2-methyl-1-pentene.

After a reaction time of 90 min. the yield of dimer was 0.80 mmol.

Example 2

In a 30 ml glass bottle, placed in an argon atmosphere, 5 mg (0.02 mmol.) of $Cp_2TiCl_2$ were weighed in, followed by 6.7 mg (0.06 mmol.) of 1,4-diaza-bicyclo[2.2.2]octane, after which 4.3 ml (34.4 mmol.) of 1-hexene and 6 ml of bromobenzene were added. The resulting solution was saturated with argon, whereupon 0.031 ml (0.3 mmol.) ethylaluminium dichloride was added to the solution. The temperature was kept on 25°C.

Gas-chromatographic analysis of the product revealed that substantially one product had been formed, which appeared to be 2-butyl-1-octene.

After a 5.5 hours' reaction time the dimer yield amounted to 2.5 mmol., which corresponds to a conversion of 14.5%.

When repeating the experiment, but without the addition of 1,4-diaza-bicyclo[2.2.2]octane, an unacceptably high amount of higher polymers appeared to have been formed.

Example 3

Following a procedure similar to that of Example 2, 5 ml (40 mmol.) of 1-hexene were dimerized in a solution containing 8 mg (0.029 mmol.) of bis-(methylcyclopentadienyl)titanium dichloride, 0.045 ml (0.44 mmol.) of ethylaluminium dichloride, 11 mg (0.10 mmol.) of 1,4-diaza-bicyclo[2.2.2]octane and 7 ml of chlorobenzene.

After 19.5 h 1.57 mmol. 2-butyl-1-octene

had been formed, which corresponds to a conversion of 7.9%.

Example 4

Following a procedure similar to that of Example 2, 5 ml (40.0 mmol.) of 4-methyl-1-pentene were dimerized in a solution containing 7.2 mg (0.029 mmol.) of $Cp_2TiCl_2$, 0.045 ml (0.44 mmol.) of ethylaluminium dichloride, 8 mg (0.072 mmol.) of 1,4-diazabicyclo[2.2.2]octane and 7 ml of chlorobenzene. The temperature was kept at 25°C.

In 2 hours' time 1.08 mmol. 2-isobutyl-6-methyl-1-heptene had been formed, which corresponds to a conversion of 5.4%.

## Claims

1. Process for the catalytic dimerization in solution of one or more alpha,beta-unsubstituted 1-alkenes having at least three carbon atoms per molecule, characterized in that a catalyst is used comprising a titanium component of the formula $Cp_2TiXY$, an alkylaluminium halide and a nitrogen Lewis base, in which formula Cp represents an unsubstituted or alkyl-substituted cyclopentadienyl group, X a halogen atom or an alkyl group and Y a halogen atom.

2. Process as claimed in claim 1, characterized in that in the formula for the titanium component X represents a chlorine atom or an alkyl group having 1—5 carbon atoms and Y represents a chlorine atom.

3. Process as claimed in claim 2, characterized in that the titanium component is dicyclopentadienyltitanium dichloride.

4. Process as claimed in any one of the preceding claims, characterized in that the alkylaluminium halide is an ethylaluminium halide.

5. Process as claimed in claim 4, characterized in that the ethylaluminium halide is ethylaluminium dichloride.

6. Process as claimed in any one of the preceding claims, characterized in that the nitrogen Lewis base is a tertiary amine.

7. Process as claimed in claim 6, characterized in that the nitrogen Lewis base is 1,4-diazabicyclo[2.2.2]octane.

8. Process as claimed in any one of the preceding claims, characterized in that the catalyst comprises at least 0.5 mmol. of titanium component per mol. of alpha,beta-unsubstituted 1-alkene.

9. Process as claimed in any one of the preceding claims, characterized in that the aluminium/titanium molar ratio in the catalyst is in the range of from 4 to 20.

10. Process as claimed in claim 9, characterized in that the aluminium/titanium molar ratio in the catalyst is in the range of from 10 to 15.

11. Process as claimed in any one of the preceding claims, characterized in that the nitrogen Lewis base is applied in such an amount that the nitrogen/aluminium molar ratio in the catalyst is higher than 0.1.

12. Process as claimed in claim 11, characterized in that the nitrogen/aluminium molar ratio in the catalyst is in the range of from 0.2 to 0.5.

13. Process as claimed in any one of the preceding claims, characterized in that the solvent used is a halogenated aromatic hydrocarbon.

14. Process as claimed in claim 13, characterized in that the solvent used is bromobenzene or chlorobenzene.

15. Process as claimed in any one of the preceding claims, characterized in that the reaction is carried out at a temperature between 10 and 40°C.

16. Process as claimed in any one of the preceding claims, characterized in that the reaction is carried out at a pressure between 100 and 500 kPa.

## Revendications

1. Procédé de dimérisation catalytique en solution d'un ou plusieurs 1-alcènes contenant au moins 3 atomes de carbone par molécule et non substitués en alpha, bèta, caractérisé en ce que l'on utilise un catalyseur qui comprend un composé du titane de formule $Cp_2$ TiXY, un halogénure d'alkyl-aluminium et une base de Lewis azotée, le symbole Cp de la formule indiquée représentant un groupe cyclopentadiényle non substitué ou alkyl-substitué, X représentant un atome d'halogène ou un groupe alkyle et Y un atome d'halogène.

2. Procédé selon la revendication 1, caractérisé en ce que, dans la formule du composé du titane, X représente un atome de chlore ou un groupe alkyle en C1—C5 et Y représente un atome de chlore.

3. Procédé selon la revendication 2, caractérisé en ce que le composé du titane est le dichlorure de dicyclopentadiényl-titane.

4. Procédé selon l'une quelconque des revendications qui précèdent, caractérisé en ce que l'halogénure d'alkyl-aluminium est un halogénure d'éthyl-aluminium.

5. Procédé selon la revendication 4, caractérisé en ce que l'halogénure d'éthyl-aluminium est le dichlorure d'éthyl-aluminium.

6. Procédé selon l'une quelconque des revendications qui précèdent, caractérisé en ce que la base de Lewis azotée est une amine tertiaire.

7. Procédé selon la revendication 6, caractérisé en ce que la base de Lewis azotée est le 1,4-diazabicyclo[2.2.2]octane.

8. Procédé selon l'une quelconque des revendications qui précèdent, caractérisé en ce que le catalyseur comprend au moins 0,5 mmole de composant du titane par mole de 1-alcène non substitué en alpha, bêta.

9. Procédé selon l'une quelconque des revendications qui précèdent, caractérisé en ce

que le rapport molaire aluminium titane dans le catalyseur se situe dans l'intervalle de 4 à 20.

10. Procédé selon la revendication 9, caractérisé en ce que le rapport molaire aluminium/titane dans le catalyseur se situe dans l'intervalle de 10 à 15.

11. Procédé selon l'une quelconque des revendications qui précèdent, caractérisé en ce que la base de Lewis azotée est appliquée en quantité telle que le rapport molaire azote/aluminium dans le catalyseur soit supérieur à 0,1.

12. Procédé selon la revendication 11, caractérisé en ce que le rapport molaire azote/aluminium dans le catalyseur se situe dans l'intervalle de 0,2 à 0,5.

13. Procédé selon l'une quelconque des revendications qui précèdent, caractérisé en ce que le solvant utilisé est un hydrocarbure aromatique halogéné.

14. Procédé selon la revendication 13, caractérisé en ce que le solvant utilisé est le bromobenzène ou le chlorobenzène.

15. Procédé selon l'une quelconque des revendications qui précèdent, caractérisé en ce que la réaction est effectuée à une température de 10 à 40°C.

16. Procédé selon l'une quelconque des revendications qui précèdent, caractérisé en ce que la réaction est effectuée à une pression de 100 à 500 kPa.

**Patentansprüche**

1. Verfahren zur katalytischen Dimerisierung eines oder mehrerer $\alpha,\beta$-unsubstituierter 1-Alkene enthaltend mindestens 3 Kohlenstoffatome je Molekül, in Lösung, dadurch gekennzeichnet, daß ein Katalysator verwendet wird, der eine Titanverbindung der Formel $Cp_2TiXY$, ein Alkylaluminiumhalogenid und eine Stickstoff-Lewis-Base umfaßt, wobei in der Formel Cp eine unsubstituierte oder alkylsubstituierte Cyclopentadienylgruppe, X ein Halogenatom oder eine Alkylgruppe und Y ein Halogenatom bedeutet.

2. Verfahren nach Anspruch 1, dadurch gekennzeichnet, daß in der Formel für die Titanverbindung X ein Chloratom odereine Alkylgruppe mit 1 bis 5 Kohlenstoffatomen und Y ein Chloratom bedeutet.

3. Verfahren nach Anspruch 2, dadurch gekennzeichnet, daß die Titanverbindung Dicyclopentadienyltitandichlorid ist.

4. Verfahren nach einem der vorangegangenen Ansprüche, dadurch gekennzeichnet, daß das Alkylaluminiumhalogenid ein Ethylaluminiumhalogenid ist.

5. Verfahren nach Anspruch 4, dadurch gekennzeichnet, daß das Ethylaluminiumhalogenid Ethylaluminiumdichlorid ist.

6. Verfahren nach einem der vorangegangenen Ansprüche, dadurch gekennzeichnet, daß die Stickstoff-Lewis-Base ein tertiäres Amin ist.

7. Verfahren nach Anspruch 6, dadurch gekennzeichnet, daß die Stickstoff-Lewis-Base 1,4-Diazabicyclo[2.2.2]octan ist.

8. Verfahren nach einem der vorangegangenen Ansprüche, dadurch gekennzeichnet, daß der Katalysator mindestens 0,5 mmol Titanverbindung je mol $\alpha,\beta$-unsubstituiertes 1-Alken umfaßt.

9. Verfahren nach einem der vorangegangenen Ansprüche, dadurch gekennzeichnet, daß das Aluminium/Titan Molverhältnis im Katalysator im Bereich von 4 bis 20 liegt.

10. Verfahren nach Anspruch 9, dadurch gekennzeichnet, daß das Aluminium/Titan Molverhältnis im Katalysator im Bereich von 10 bis 15 liegt.

11. Verfahren nach einem der vorangegangenen Ansprüche, dadurch gekennzeichnet, daß die Stickstoff-Lewis-Base in einer solchen Menge eingesetzt wird, daß das Stickstoff/Aluminium Molverhältnis im Katalysator über 0,1 liegt.

12. Verfahren nach Anspruch 11, dadurch gekennzeichnet, daß das Stickstoff/Aluminium Molverhältnis im Katalysator im Bereich von 0,2 bis 0,5 liegt.

13. Verfahren nach einem der vorangegangenen Ansprüche, dadurch gekennzeichnet, daß das eingesetzte Lösungsmittel ein halogenierter aromatischer Kohlenwasserstoff ist.

14. Verfahren nach Anspruch 13, dadurch gekennzeichnet, daß das eingesetzte Lösungsmittel Brombenzol oder Chlorbenzol ist.

15. Verfahren nach einem der vorangegangenen Ansprüche, dadurch gekennzeichnet, daß die Reaktion bei einer Temperatur zwischen 10 und 40°C ausgeführt wird.

16. Verfahren nach einem der vorangegangenen Ansprüche, dadurch gekennzeichnet, daß die Reaktion bei einem Druck zwischen 100 und 500 kPa ausgeführt wird.